# EUROPEAN PATENT APPLICATION

(11) **EP 0 730 035 A1**
(43) Date of publication of application: **04.09.1996**
(21) Application number: 95120490.8
(22) Date of filing: 22.12.1995
(51) Int. Cl.: C12P 35/04, C12P 37/04, C12P 17/18, C12N 11/08

(54) **Improved enzymatic process for producing penicillins and cephalosporins**

(30) Priority: 28.02.1995 IT MI950383
(71) Applicant: ACS DOBFAR S.p.A., I-20067 Tribiano, Milan (IT)
(72) Inventor: Zenoni, Maurizio, I-20067 Paullo (IT)
(74) Representative: Frignoli, Luigi

(57) **Abstract**

A process for producing penicillins or cephalosporins by reacting a 6-amino penicillanic acid or 7-amino cephalosporanic acid in aqueous medium with an amide in the presence of a penicillin acylase enzyme immobilized on an azlactone polymer.

## Description

The present invention relates to an improved enzymatic process for producing penicillins and cephalosporins of formulae (I) and (II) 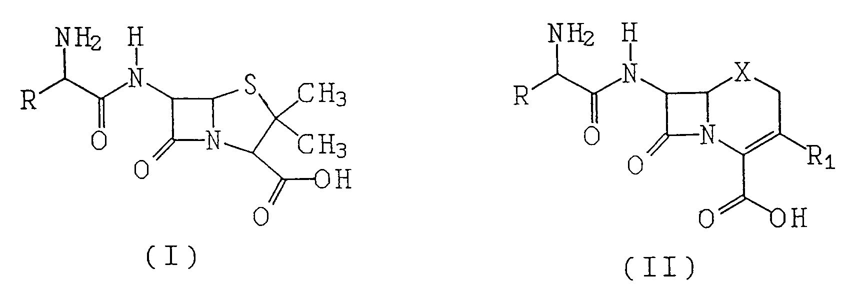 wherein X is S or CH₂, R is a six-membered hydrocarbon ring optionally substituted and R₁ is a hydrogen atom, a halogen atom, a methyl group, a methoxy group, an alkenyl C₁-C₄ group or a methylene group bonded to an organic radical via an atom of oxygen, sulphur or nitrogen.

US-A-3816253 discloses a process for producing a penicillin or cephalosporin by the reaction of an a-substituted a-aminoacid and a compound derived from 7-aminocephalosporanic acid or 7-aminodeacetoxy-cephalosporanic acid in the presence of an active microorganism or enzyme in an aqueous medium at a temperature between +5°C and +50°C, in particular in the range of +20°C to +40°C. It has been found that when operating as suggested by the cited US patent the yield of the desired final product is greatly reduced by concurrent parallel reactions causing the formation of by-products which are particularly difficult to separate from the reaction mixture.

In order to overcome or reduce the above mentioned drawbacks, the EP-A-0473008 proposed a method for producing a penicillin or cephalosporin of formula (I) or (II) according to which a 6-aminopenicillanic or 7-aminocephalosporanic acid is reacted with an a-substituted a-aminoacid of formula R-CH(NH₂)-COOH or a reactive derivative thereof in the presence of an immobilized penicillin acylase, the reaction being carried out in aqueous medium at temperatures between -5°C and +20°C, preferably at about +4°C.

All the reaction conditions are clearly given in the specification of EP-A-0473008 which gives also a series of 25 detailed examples showing a very good yield (up to 90% or more) of the desired final product.

In this connection it is to be noted that a process is industrially workable when the yields are sufficiently high (about 90%) and when the final product can be easily purified.

EP-A-0473008 mentions in generic terms the a-substituted a-aminoacids of formula R-CH(NH₂)-COOH and their reactive derivatives which may be used as reagents but the only aminoacid specifically exemplified is the methyl ester of D-phenylglycine.

Very accurate and repeated tests have been carried out by the present applicants following the procedures disclosed in the examples of the EP-A-0473008 but the results were discouraging. Indeed it has been found that if the molar ratio of the cited aminoacid in the reaction medium is less than 4 moles of the aminoacid per 1 mole of the 6-amino penicillanic or 7-amino cephalosporanic acid the yield of the final desired compound is very low (lower than about 60%) and, as such, not industrially acceptable.

Good and acceptable yields (about 90%) can be obtained only if the number of moles of the D-phenylglicine methyl ester is between 4 and 6 per 1 mole of the 6-aminopenicillanic or 7-aminocephalosporanic acid. However, in this case there is not only an unacceptable increase in costs (the price of the D-phenylglycine methyl ester is high) but by-product are formed which are very difficult or impossible to separate from the desired final penicillin or cephalosporin.

Attempts have been made to use other types of esters of the D-phenylglycine or of other aminoacids, but all efforts have been unsuccessful. The same negative results were obtained using a lot of other reactive derivatives of the aminoacid.

The German patent application DOS 2214444 describes the enzimatic synthesis of cephalosporins (in particular cephalexin) by reacting 7-ADCA with phenylglycine amide in the presence of a penicillin acylase enzyme: the obtained yields are very low.

PCT/DK91/00188 (WO92/01061) and PCT/DK92/00388 (WO93/12250) disclose methods for the preparation of b-lactam antibiotics by enzymatic acylation, according to which an amide of formula (III) 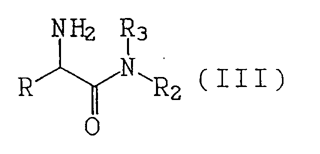 is reacted with a 6-amino-penicilanic acid or 7-amino-cephalosporanic acid in the presence of an immobilised penicillin acylase enzyme. Such methods have proved to be workable, but it has been found that an appreciable amount of the amide (III) is hydrolized by the same enzyme, thus increasing the impurity of the desired final products: also a portion of the final products is hydrolized by the enzyme.

The present applicants have carried out several experiments following the teachings of the two above quoted PCT cases, while using different amides as acylating agents, different enzymes as bacterial sources and different solid matrices for the immobilisation of the enzymes.

The results which have been obtained have been comparable to each other, showing always an appreciable hydrolysis of both the amides and the final products.

It has been surprisingly found that following the same procedures disclosed in said WO92/01061 and WO93/12250 but immobilizing the enzymes on a particular support which is an azlactone polymer (a per se know polymer commonly used as a biosupport medium for affinity chromatography and sold by Minnesota Mining and Manufacturingg Co. under the Trade name EMPHAZE) the hydrolysis of the amides (III) and of the final products are substantially reduced. Such a reduction can be quantified in the order of about 30%, what is important not only from the point of view of process economy but especially because the final products can be obtained with higher purities and also because the risk of having crystallization of the hydrolized product (phenylglycine and its derivatives) is avoided. In this connection it is to be noted that crystallization can easily take place while using all other immobilising matrices which have been tested.

As a consequence, the present invention concerns an improved enzymatic process for producing penicillins and cephalosporins of formulae (I) and (II) as specified hereabove, in which a 6-amino-penicillanic acid or 7-amino-cephalosporanic acid of formula (IV) or (V) 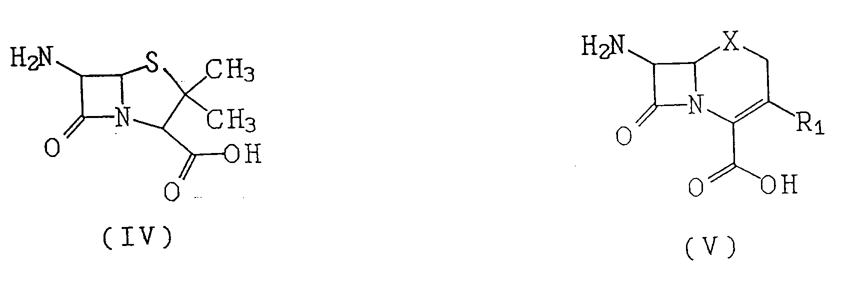 wherein X and R₁ are as defined above, is reacted in an aqueous medium at a temperature from -5°C to +35°C and in the presence of an immobilised penicillin acylase enzyme with an amide of formula (III) 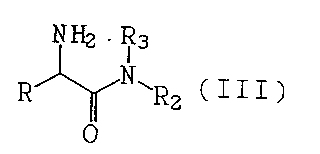 wherein R is as specified above, and R₂ and R₃ are each independently hydrogen or a linear or branched alkyl group of 1 to 3 carbon atoms or salt thereof, in a molar ration from 1.5 to 3 moles of said amide (III) per 1 mole of said acid (IV) or (V), characterized in that said enzyme is immobilised on an azlactone polymer.

In particular said amide (III) is D-phenylglycine amide or a salt thereof with an organic or inorganic acid.

The invention relates also to the pharmaceutical compositions comprising penicillin or cephalosporin prepared by the process according to the present invention, as well as a pharmaceutical acceptable carrier or diluent.

The pharmaceutical compositions containing the penicillin or cephalosporin are usually prepared following conventional methods and are administered in a pharmaceutically suitable form.

With respect to the formulae (I) and (II), R may be, for example, phenyl, cyclohexadienyl, cyclohexenyl or cyclohexyl, either unsubstituted or substituted by one or more hydroxyl, halogen, alkyl,alkoxy, carboxyl, nitro or amino.

R¹, in turn, can be a hydrogen atom, a halogen atom, a methyl group or a methylene group bonded to an organic group particularly an alkoxy, an alkoxycarbonyl or to a five or six-membered heterocyclic group containing 1 to 4 heteroatoms selected from O, S and N, bonded to the methylene group via an atom of O, S or N, and optionally bearing as substituents one or more groups selected among: hydroxy, halogen, alkyl, alkoxy, carbonyl, carboxy, cyano and amino and the like.

The terms "alkyl" and "alkoxy" as used herein indicate groups of 1 to 6 carbon atoms, preferably 1 to 4 carbon atoms.

With respect to the a-aminoacids whose amide has the formula (III), as examples can be mentioned D-phenylglycine, D-p-hydroxyphenylglycine, and D-1,4-cyclohexadien-1-yl-glycine.

Suitable salts of the amide are with an inorganic acid such a hydrochloric, hydrofluoric, hydrobromic, sulphuric or nitric acid, or with an organic acid such as acetic, formic or maleic acid.

The following acids are of particularly interest among the compounds of formula (IV) and (V): - 6-amino-penicillanic acid, 7-amino-cephalosporanic acid, 7-amino-3-deacetoxy-cephalosporanic acid, 7-amino-3-chloro-cephalosporanic acid.

The enzyme used in the present process may be a penicillin acylase enzyme or penicillin amidase enzyme. The penicillin acylase or amidase enzyme may derive from any of the known microbial sources. Among these mention is made in particular of microorganisms of the Xanthomonas, Pseudomonas, Aeromonas, Escherichia, Arthrobacter, Acetobacter, Mycoplasma protaminobacter, Kluyvera, Corynbacterium and Bacillus genera. Enzymes derived from E. coli are particularly preferred since they are easily and cheaply available commercially in free or immobilized forms.

Penicillin acylase or amidase enzymes classified as EC 3.5.1.11 may in particular be used.

The following Examples illustrate the invention.

### EXAMPLE 1

### Penicillin G acylase immobilization on azalactone functional support.

138.8 mg of a penicillin acylase in 2ml of a solution of 0.1M phosphate at pH 7.5 was mixed with 28ml of a coupling buffer at pH 7.4 with 1.1M sodium sulphate and 0.1M sodium phosphate. 1g of the azalactone functional support (EMPHAZE™) was added into a 50ml centrifuge tube. The enzyme in coupling buffer was added to the dry beads and mixed on a rocker platform, end over end, for 3 hours at room temperature. The excess coupling buffer solution was drained, and the beads washed 5 times with 10 ml aliquots of PBS (20 mM phosphate, 0,9% sodium chloride at pH 7.4).This resulted in >90% of the enzyme activity after immobilization unto this support.

### EXAMPLE 2

### Cefaclor with b-naphtol present in enzyme reactor.

11.69g (49.82 mmoles) of 3-chloro-7-aminodesacetoxycephalosporanic acid (3Cl/Nu) and 15.22g (101.47 mmoles) of D-phenylglycine amide (FGA) were dissolved in H₂O for a total volume of 196ml. Then, 8g (55.44 mmoles) of finely ground b-naphtol were added to that solution in the same reactor. The acylation was carried out with 21,2g (3700 I.U.) of penicillin G amidase, prepared as in example 1, (PGA) in the following conditions: pH was held stable at 6.8 with 4N sulphuric acid at a temperature of 10°C. After about 2 hours, 98% of 3Cl/Nu was transformed to cefaclor [7-(D-2-amino-2-phenylacetamido)-3-chloro-3-cephem-4-carboxylic acid] complexed with b-naphtol. After 98% conversion of 3Cl/Nu, the molar-ratio selectivity of the synthetic production of cefaclor to the hydrolytic production of D-phenylglycine was 4.12 compared to 3.56 with PGA supported on polyacrylic oxirane beads.

### EXAMPLE 3

### Cefaclor complexed with b-naphtol continuously in a separate crystallisation reactor

47.71g (203.37 mmoles) of 3Cl/Nu and 87.3g (582.53 mmoles) of FGA were dissolved in H₂O for a total volume of 1070ml. Then, 30g (207.90 mmoles) of finely ground b-naphtol were added in the crystallisation reactor. The solution was recycled through the enzymation reactor, where the acylation was carried out with 25g (4500 I.U.) of PGA, to the crystallisation reactor. The complex product was filtered out and the mother liquor was returned to the enzymation reactor to complete the conversion. The process was performed with the following conditions: pH was held stable at 6.8 with 4N sulphuric acid at a temperature of 10°C. After about 3 hours of recycling, 94% of 3Cl/Nu was transformed to cefaclor, complexed with b-naphtol in the crystallisation reactor. After 94% conversion of 3Cl/Nu, the molar-ratio selectivity of the synthetic production of cefaclor to the hydrolytic production of D-phenylglycine was 3.05 compared to 2.37 with PGA supported on polyacrylic oxiranic beads.

### EXAMPLE 4

### Cefaclor dihydrate crystallised continuously in a separate reactor.

Same initial solution as example 3. The solution was recycled through the enzymation reactor, where the acylation was carried out with 25g (4500 I.U.) of PGA, to the crystallisation reactor, where the pH was lowered to 6.4 in order to promote precipitation of cefaclor.2H₂O. The crystallised product was filtered out and the mother liquor was returned to the enzymation reactor to complete the conversion. The process was performed with the following conditions: pH was held stable at 6.4 with 4N sulphuric acid in the crystallisation reactor at a temperature of 2°C for both reactors. After about 3 hours of recycling, 93% of 3Cl/Nu was transformed to cefaclor.2H₂O in the crystallisation reactor. After 93% conversion of 3Cl/Nu, the molar-ratio selectivity of the synthetic production of cefaclor to the hydrolytic production of D-phenylglycine was 2.61 compared to 2.02 with PGA supported on polyacrylic oxirane beads.

### EXAMPLE 5

### Acylation of 7-ADCA to cephalexin with FGA in a doubte step enzymation and crystallisation.

25.05g (117.05 mmoles) of 7-aminodesacetoxycephalosporanic acid (7-ADCA), 30.05g (198.99 mmoles) of FGA were dissolved in H₂0 for a total volume of 498 ml. The acylation was carried out with 21.0g (3750 I.U.) of PGA in the following conditions: initial pH of first step was 6.57 a temperature of 3°C. After about 1 hour, 61.9% of 7-ADCA was transformed to cephalexin [7-(D-2-amino-2-phenylacetamide)-3-methyl-3-cephem-4-carboxyl acid]. The majority of the cephalexin was isolated from the rich liquor complexing it with 11.50g (79.70 mmoles) of b-naphtol. The second step of the reaction was then completed by treating the mother liquor with the same PGA as above. After about 2 hours, 94,5% of 7-ADCA was transformed to cephalexin, totally after both steps. The majority of the cephalexin was isolated from the rich liquor of the second step complexing it with 7.50g (51.98 mmoles) of b-naphtol . After 94.5% conversion of 7-ADCA, the molar-ratio selectivity of the synthetic production of cefalexin to the hydrolytic production of D-phenylglycine was 2.27 compared to 1.53 with PGA supported on polyacrylic oxirane beads.

### EXAMPLE 6

### Acylation of 7-amino-3-chloro-carbacephem acid to Loracarbef with FGA.

3.32g (15.31 mmoles) of 3-chloro-7-aminocarbacephem acid (3Cl/CNu) and 8.65g (57.70 mmoles) of FGA were dissolved in H₂O for a total volume of 190ml. The acylation was carried out with 5g (895 I.U.) of PGA in the following conditions: pH was held stable at 6.2 with 1N hydrochloric acid at a temperature of 3°C. After about 2 hours, 89.5% of 3Cl/CNu was transformed to loracarbef [7-(D-2-amino-2-phenylacetamido)-3-chloro-3-carbacephem-4- carboxylic acid]. After 89.5% conversion of 3Cl/CNu, the molar-ratio selectivity of the synthetic production of loracarbef to the hydrolytic production of D-phenylglycine was 2,76 compared to 2.15 with PGA supported on polyacrylic oxirane beads.

## Claims

1. A process for producing a penicillin or cephalosporin of formula (I) or (II) 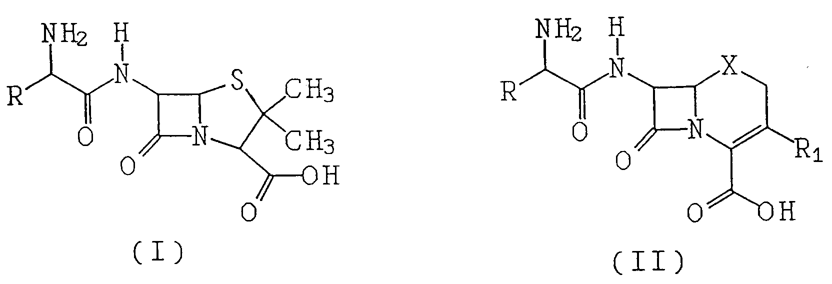 wherein X is S or CH₂, R is an optionally substituted six-membered hydrocarbon ring and R₁ is a hydrogen atom, a halogen atom, a methyl group, a methoxy group, an alkenyl C₁-C₄ group or a methylene group bonded to an organic radical via an atom of oxygen, sulphur or nitrogen; in which: a 6-amino-penicillanic acid or 7-amino-cephalosporanic acid of formula (IV) or (V) 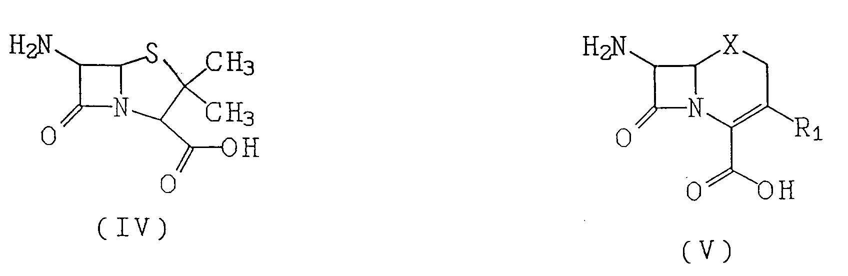 wherein X and R₁ are as defined above, is reacted in an aqueous medium at a temperature from -5°C to +35°C and in the presence of an immobilised penicillin acylase enzyme with an amide of formula (III) 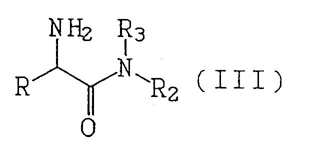 wherein R is as specified above, and R₂ and R₃ are each independently hydrogen or a linear or branched alkyl group of 1 to 3 carbon atoms or salt thereof, in a molar ratio from 1.5 to 3 moles of said amide (III) per 1 mole of said acid (IV) or (V), characterized in that said enzyme is immobilised on an azlactone polymer.

2. A process according to claim 1, characterized in that said amide (III) is D-phenylglycine amide or a salt thereof with an organic or inorganic acid.

3. A pharmaceutical composition comprising penicillin or cephalosporin prepared by a process according to claims 1 and 2 and pharmaceutical acceptable carrier or diluent.

4. An enzyme penicillin acylase immobilized on azlactone polymer.
